# EUROPEAN PATENT SPECIFICATION

(11) **EP 0 853 671 B1**
(45) Date of publication and mention of the grant of the patent: **26.01.2005**
(21) Application number: 96933212.1
(22) Date of filing: 01.10.1996
(51) Int. Cl.: C12N 15/32, C07K 14/325, C12Q 1/68, A01N 63/00

(54) **IDENTIFICATION OF, AND USES FOR, NEMATICIDAL BACILLUS THURINGIENSIS GENES, TOXINS, AND ISOLATES**
IDENTIFIZIERUNG UND VERWENDUNGEN VON NEMATIZIDE BACILLUS THURINGIENSIS GENE, TOXINE UND ISOLATE
IDENTIFICATION ET UTILISATIONS D'ISOLATS, DE TOXINES ET DE GENES NEMATICIDES DE BACILLUS THURINGIENSIS

(30) Priority: 06.10.1995 US 540104; 21.03.1996 US 620717
(43) Date of publication of application: 22.07.1998
(73) Proprietor: Mycogen Corporation, San Diego, CA 92121 (US)
(72) Inventor: FEITELSON, Jerald, S., San Diego, CA 92130 (US)
(74) Representative: Perry, Robert Edward
(86) International application number: PCT/US1996/015730
(87) International publication number: WO 1997/012980

(56) References cited:
- EP-A- 0 462 721
- WO-A-92/19739
- WO-A-92/20802
- US-A- 5 430 137
- APPLIED AND ENVIRONMENTAL MICROBIOLOGY, vol. 57, no. 11, November 1991, pages 3057-3061, XP000644429 CAROZZI, N. ET AL.: "Prediction of insecticidal activity of Bacillus thuringiensis strains by polymerase chain reaction product files" cited in the application

## Description

### Background of the Invention

The soil microbe *Bacillus thuringiensis* (*B*.*t*.) is a Gram-positive, spore-forming bacterium characterized by parasporal crystalline protein inclusions. These inclusions often appear microscopically as distinctively shaped crystals. The proteins can be highly toxic to pests and specific in their toxic activity. Certain *B*.*t*. toxin genes have been isolated and sequenced, and recombinant DNA-based *B*.*t*. products have been produced and approved for use. In addition, with the use of genetic engineering techniques, new approaches for delivering these *B*.*t*. endotoxins to agricultural environments are under development, including the use of plants genetically engineered with endotoxin genes for insect resistance and the use of stabilized intact microbial cells as *B*.*t*. endotoxin delivery vehicles (Gaertner *et al*., 1988). Thus, isolated *B*.*t*. endotoxin genes are becoming commercially valuable.

Until the last ten years, commercial use of *B*.*t*. pesticides has been largely restricted to a narrow range of lepidopteran (caterpillar) pests. Preparations of the spores and crystals of *B*. *thuringiensis* subsp. *kurstaki* have been used for many years as commercial insecticides for lepidopteran pests. For example, *B. thuringiensis* var. *kursraki* HD-1 produces a crystalline δ-endotoxin which is toxic to the larvae of a number of lepidopteran insects.

In recent years, however, investigators have discovered *B*.*t*. pesticides with specificities for a much broader range of pests. For example, other species of *B*.*t*., namely *israelensis* and *morrisom* (a.k.a. *tenebrionis*, a.k.a. *B*.*t*. M-7, a.k.a. *B*.*t*. *san diego*), have been used commercially to control insects of the orders Diptera and Coleoptera, respectively (Gaertner, 1989). See also Couch, 1980 and Beegle, 1978. Krieg *et al*., 1983, describe *Bacillus thuringiensis* var. *tenebrionis*, which is reportedly active against two beetles in the order Coleoptera. These are the Colorado potato beetle, *Leptinotarsa decemlineata,* and *Agelasnca alni*.

Recently, new subspecies of *B. t*. have been identified, and genes responsible for active δ-endotoxin proteins have been isolated (Höfte and Whiteley, 1989). Höfte and Whiteley classified *B. t.* crystal protein genes into four major classes. The classes were CryI (Lepidoptera-specific), CryII (Lepidoptera- and Diptera-specific), CryIII (Coleoptera-specific), and CryIV (Diptera-specific). The discovery of strains specifically toxic to other pests has been reported. (Feitelson *et al*., 1992). CryV has been proposed to designate a class of toxin genes that are nematode-specific.

The cloning and expression of a *B*.*t*. crystal protein gene in *Escherichia coli* has been described in the published literature (Schnepf and Whiteley, 1981). U.S. Patent 4,448,885 and U.S. Patent 4,467,036 both disclose the expression of *B*.*t*. crystal protein in *E*. *coli*. U.S. Patents 4,990,332; 5,039,523; 5,126,133; 5,164,180; and 5,169,629 are among those which disclose *B*.*t*. toxins having activity against lepidopterans. U.S. Patents 4,797,276 and 4,853,331 disclose *B*. *thuringiensts* strain *tenebrionis* which can be used to control coleopteran pests in various environments. U.S. Patent No. 4,918,006 discloses *B*.*t*. toxins having activity against dipterans. U.S. Patent No. 5,151,363 and U.S. Patent No. 4,948,734 disclose certain isolates of *B*.*t*. which have activity against nematodes. Other U.S. Patents which disclose activity against nematodes include 5,093,120; 5,236,843; 5,262,399; 5,270,448; 5,281,530; 5,322,932; 5,350,577; 5,426,049; and 5,439,881. As a result of extensive research and investment of resources, other patents have issued for new *B*.*t*. isolates and new uses of *B*.*t*. isolates. See Feitelson *et al*., 1992 for a review. However, the discovery of new *B*.*t*. isolates and new uses of known *B*.*t*. isolates remains an empirical, unpredictable art.

Regular use of chemical control of unwanted organisms can select for chemical resistant strains. Chemical resistance occurs in many species of economically important insects and has also occurred in nematodes of sheep, goats, and horses. The development of chemical resistance necessitates a continuing search for new control agents having different modes of action. The subject invention pertains specifically to materials and methods for the identification of *B*. *t*. toxins active against nematodes or coleopteran pests. Of particular interest among the coleopteran pests is the corn rootworm.

In recent times, the accepted methodology for control of nematodes has centered around the drug benzimidazole and its congeners. The use of these drugs on a wide scale has led to many instances of resistance among nematode populations (Prichard *et al*., 1980; Coles, 1986). There are more than 100,000 described species of nematodes.

A small number of research articles have been published about the effects of delta endotoxins from *B*. *thuringiensis* species on the viability of nematode eggs. Bottjer, Bone and Gill, (1985) have reported that *B*.*t*. *kurstaki* and *B*.*t*. *israelensis* were toxic in vitro to eggs of the nematode *Trichostrongylus colubriformis*. In addition, 28 other *B*.*t*. strains were tested with widely variable toxicities. Ignoffo and Dropkin, 1977, have reported that the thermostable toxin from *Bacillus thuringiensis* (beta exotoxin) was active against a free-living nematode, *Panagrellus redivivus* (Goodey); a plant-parasitic nematode, *Meloidogyne incognita* (Chitwood); and a fungus-feeding nematode, *Aphelenchus avena* (Bastien). Beta exotoxin is a generalized cytotoxic agent with little or no specificity. Also, Ciordia and Bizzell (1961) gave a preliminary report on the effects of *B*. *thuringiensis* on some cattle nematodes.

There are a number of beetles that cause economic damage. Corn rootworms include species found in the genus *Diabrotica* (*e*.*g*., *D. undecimpunctata undecimpunctata, D. undecimpunctata howardii, D. longicornis, D. virgifera* and *D. balteata*). Corn rootworms cause extensive damage to corn and curcubits. Approximately $250 million worth of insecticides are applied annually to control corn rootworms alone in the United States. Even with insecticide use, rootworms cause about $750 million worth of crop damage each year, making them the most serious corn insect pest in the Midwest.

Current methods for controlling corn rootworm damage in corn are limited to the use of crop rotation and insecticide application. However, economic demands on the utilization of farmland restrict the use of crop rotation. In addition, an emerging two-year diapause (or overwintering) trait of Northern corn rootworms is disrupting crop rotations in some areas.

The use of insecticides to control corn rootworm and other coleopteran pests also has several drawbacks. Continual use of insecticides has allowed resistant insects to evolve. Insecticide use often raises environmental concerns such as contamination of soil and of both surface and underground water supplies. Working with insecticides may also pose hazards to the persons applying them.

At the present time there is a need to have more effective means to control the many nematodes and coleopterans that cause considerable damage to susceptible hosts and crops. Advantageously, such effective means would employ specific biological agents.

*Bacillus thuringiensis* toxins which are active against nematodes and corn rootworm are now known. Isolating responsible toxin genes has been a slow empirical process. Carozzi *et al*., 1991 describe methods for identifying toxin genes. This report does not disclose or suggest the specific primers and probes of the subject invention for nematode-active and corn rootworm-active toxin genes. U.S. Patent No. 5,204,237 describes specific and universal probes for the isolation of *B*. *t*. toxin genes. This patent, however, does not describe the probes and primers of the subject invention.

### Summary of the Invention

A method for controlling nematodes, according to the invention, comprises contacting the nematodes with a nematicidal toxin obtainable from *Bacillus thuringiensis* isolate PS86Q3, or a nematicidal fragment of said toxin.

### Description of the Invention

Isolate PS86Q3 has a crystal description "attached long". Its approximate molecular weight is 155, 135, 98, 62, 58 kDa. It is available from the permanent collection of the Agricultural Research Service Plant Culture Collection (NRRL), Northern Regional Research Center, 1815 North University Street, Peoria, Illinois 61604, USA. The deposit number is B-18765. The deposit date is 6 June 1991.

The nematicidal toxin known as 86Q3(a) found in PS86Q3, is encoded by a gene described in WO92/20802.

*B*.*t*. isolates can be cultivated under conditions resulting in high multiplication of the microbe. After treating the microbe to provide single-stranded genomic nucleic acid, the DNA can be contacted with the primers, and subjected to PCR amplification. Characteristic fragments of toxin-encoding genes will be amplified by the procedure, thus identifying the presence of a toxin-encoding gene.

The sequences of five *cryV*-specific primers useful according to the subject invention are shown in Table 1.

**Table 1**

| **Primer Name** | **Sequence** |
|---|---|
| V3 | GATCGTMTWGARTTTRTTCC |
| V5 | AAAGTNGATGCMTTATCWGATGA |
| V7 | ACACGTTATAHDGTTTCTGG |
| ΔV5' | TCATCWGATAAKGCATCNAC |
| ΔV8' | TGGACGDTCTTCAMKAATTTCYAAA |

Control of nematodes according to the invention can be accomplished by a variety of methods known to those skilled in the art. These methods include, for example, the application of the B.t. isolate to the pests (or their location), the application of recombinant microbes to the pests (or their locations), and the transformation of plants with genes which encode the pesticidal toxins of the subject invention. The recombinant microbe may be, for example, a *B.t., E. coli*, or *Pseudomonas*. Transformations can be made by those skilled in the art using standard techniques. Materials necessary for these transformations are disclosed therein or are otherwise readily available to the skilled artisan.

One aspect of the subject invention is the transformation of plants with genes encoding a toxin active against coleopteran and/or nematode pests. The transformed plants are resistant to attack by coleopterans and/or nematodes.

Genes encoding pesticidal toxins, as disclosed herein, can be modified for optimum expression in plant, linked to a plant selectable marker gene, and inserted into a genome of plant cell using a variety of techniques which are well known to those skilled in the art. Any plant may be used in accordance with this invention, including angiosperms, gymnosperms, monocotyledons and dicotyledons. Preferred plants include soybean, sunflower, cotton, potato, alfalfa, maize, rice and wheat. The transformation method itself is not critical to the invention but may include transformation with T-DNA using *Agrobacterium tumefactens* or *A*. *rhizogenes* as the transformation agent, liposome fusion, microinjection, microprojectile bombardment, chemical agent (PEG or calcium chloride)-assisted DNA uptake, or electroporation, as well as other possible methods. Reference may be made to the literature for full details of the known methods, especially Holsters *et al*., 1978; Fromm *et al*., 1985; Horsch *et al*., 1985; Herrera-Estrella *et al*., 1983; Crossway *et al*., 1986; Lin, 1966; and Steinkiss and Stabel, 1983.

Use of a plant selectable marker in transformation allows for selection of transformed cells rather than cells that do not contain the inserted DNA. Various markers exist for use in plant cells and generally provide resistance to a biocide or antibiotic, including but not limited to, kanamycin, G418, hygromycin, and phosphinothricin. Visual markers including but not limited to b-glucuronidase, b-galactosidase, B-peru protein, green fluorescent protein, and luciferase may also be used. After transformation, those cells that have the DNA insert can be selected for by growth in a defined medium and assayed for marker expression, whether by resistance or visualization. Cells containing the DNA insert can be regenerated into plants. As long as stably transformed plants are obtained, the method used for regeneration will depend on the plant tissue and transformation method used and is not critical to the invention. However, for example, where cell suspensions have been used for transformation, transformed cells can be induced to produce calli and the calli subsequently induced to form shoots, which may then be transferred to an appropriate nutrient medium to regenerate plants. Alternatively, explants such as hypocotyl tissue or embryos may be transformed and regenerated by shoot induction in the appropriate media, followed by root and whole plant formation. Whatever regeneration method is used, the result will be stably transformed plants that can vegetatively and sexually transmit the transformed trait(s) to progeny, so that, if necessary, the transformed plant can be crossed with untransformed plants in order to transfer the trait to more appropriate germplasm for breeding purposes.

Following are examples which illustrate procedures for practicing the invention. These examples should not be construed as limiting. All percentages are by weight and all solvent mixture proportions are by volume unless otherwise noted.

### Example 1- Culturing of B.t. Isolates Useful According to the Invention

A subculture of *B*.*t*. isolates, or mutants thereof, can be used to inoculate the following peptone, glucose, salts medium:

| | |
|---|---|
| Bacto Peptone | 7.5 g/l |
| Glucose | 1.0 g/l |
| KH₂PO₄ | 3.4 g/l |
| K₂HPO₄ | 4.35 g/l |
| Salt Solution | 5.0 ml/l |
| CaCl₂ Solution | 5.0 ml/l |
| pH 7.2 | |

| Salts Solution (100 ml) | |
|---|---|
| MgSO₄-7H₂O | 2.46 g |
| MnSO₄-H₂O | 0.04 g |
| ZnSO₄-7H₂O | 0.28 g |
| FeSO₄-7H₂O | 0.40 g |

| CaCl₂ Solution (100 ml) | |
|---|---|
| CaCl₂·2H₂O | 3.66 g |

The salts solution and CaCl₂ solution are filter-sterilized and added to the autoclaved and cooked broth at the time of inoculation. Flasks are incubated at 30°C on a rotary shaker at 200 rpm for 64 hr.

The above procedure can be readily scaled up to large fermentors by procedures well known in the art.

The *B*.*t*. spores and/or crystals, obtained in the above fermentation, can be isolated by procedures well known in the art. A frequently-used procedure is to subject the harvested fermentation broth to separation techniques, *e*.*g*., centrifugation.

### Example 2 - Nematode Bioassay

*C*. *elegans* eggs were purified from gravid adult hermaphrodites, followed by hatching, feeding *B*.*t*.-based materials to a population of L 1 or L2 larvae, followed by a 3-day bioassay time. Hatched L1 larvae remain viable at 15 °C for more than two weeks and neither grow nor die in the absence of a bacterial food source. *C*. *elegans* adapt rapidly to a diet of non-toxic *B*.*t*. spores (and crystals), although they grow more slowly than on their usual bacterial food source, *E*. *coli*.

This bioassay was validated by feeding approximately 100 L1 or L2 C *elegans* larvae in 300 µl bioassay volume in 24-well microtiter trays with 4 doses (1, 5, 10, or 25 µl of washed biomass) of the following bacterial cells:

| | |
|---|---|
| *B*.*t*. PS17 | known nematicidal isolate |
| *B*.*t*. PS80JJ1 | known nematicidal isolate |
| *B*.*t*. PS167P | known nematicidal isolate |
| *B*.*t*. HD-73 | negative control strain |
| *E. coli strain* MC1061 | normal *C*. *elegans* food source |
| No bacteria | |

All nematodes were dead after 3 days at room temperature when incubated with the three nematicidal isolates, but proceeded to their full-grown adult stage with HD-73 or MC1061. After 5 days, L2 progeny were observed along with fully-grown adults on high doses of MC1061; HD-73 yielded only fully grown adults during this time.

Thus, all 3 known nematicidal isolates killed all nematodes, even at the lowest rate tested, while the non-toxic *B*.*t*. strain permitted full population growth without progeny interference even at the highest rate tested.

### Example 3 - Isolation and Preparation of Cellular DNA for PCR

DNA can be prepared from cells grown on Spizizen's agar, or other minimal agar known to those skilled in the art, for approximately 16 hours. Spizizen's casamino acid agar comprises 23.2 g/l Spizizen's minimal salts [(NH₄)₂SO₄, 120 g; K₂HPO₄, 840 g; KH₂PO₄, 360 g; sodium citrate, 60 g; MgSO₄·7H₂O, 12 g. Total: 1392 g]; 1.0 g/l vitamin-free casamino acids; 15.0 g/l Difco agar. In preparing the agar, the mixture was autoclaved for 30 minutes, then a sterile, 50% glucose solution can be added to a final concentration of 0.5% (1/100 vol). Once the cells are grown for about 16 hours, an approximately 1 cm² patch of cells can be scraped from the agar into 300 µl of 10 mM Tris-HCl (pH 8.0)-1 mM EDTA. Proteinase K was added to 50 µg/ml and incubated at 55°C for 15 minutes. Other suitable proteases lacking nuclease activity can be used. The samples were then placed in a boiling water bath for 15 minutes to inactivate the proteinase and denature the DNA. This also precipitates unwanted components. The samples are then centrifuged at 14,000 x g in an Eppendorf microfuge at room temperature for 5 minutes to remove cellular debris. The supernatants containing crude DNA were transferred to fresh tubes and frozen at -20°C until used in PCR reactions.

### Example 4 - PCR Amplification

Conditions for multiplex PCR amplification were:

**Table 2.**

| PCR amplification conditions | |
|---|---|
| Reagent | Final reaction concentration |
| Taq buffer | 1x |
| MgCl₂ | 2.0 mM |
| dNTPs | 0.1 mM |
| rRNA primers (forward & reverse) | 0.05 pmol/µl each |
| *cryV*-specific primers (forward & reverse) | 0.20 pmol/µl each |
| crude total *B*.*t*. DNA¹ | 15 µl |

| | |
|---|---|
| ¹Total reaction volume: 49.5 µl. | |

Samples were preheated to 94°C for 3 minutes, then quick chilled on ice. 0.5 µl Taq polymerase (5 units/ml) was added and overlaid with 50 µl light mineral oil. Cycle conditions were: {94°C, 1 minute; 42°C, 2 minutes; 72°C, 3 minutes + 5 second/cycle}, repeated for 30 cycles, and held at 4°C or -20°C until gel analysis.

Internal positive controls for each PCR reaction in the screen were included: forward and reverse 16S rRNA gene primers, yielding a PCR-amplified fragment of 182 bp corresponding to nucleotide positions 1188 to 1370 in the sequence (Ash, C. *et al*. [1991] *Lett*. *Appl*. *Microbiol*. 13:202-206). This size is smaller than fragments expected from any of the *cryV*-specific primer pairs. The two rRNA primers were:

**Table 3.**

| **PCR amplification for nematode-active *B*.*t*. strains** | | | | | | |
|---|---|---|---|---|---|---|
| | | | **Expected size (bp) using primer pair** | | | |
| **Strain** | **Gene** | **Gene name** | **V3-ΔV5'** | **V3-ΔV8'** | **V7-ΔV8'** | **V5-ΔV8'** |
| PS86Q3 | 86Q3a | *cryVD* | 562 | 1124 | 317 | 582 |
| PS86Q3 | 86Q3c | *cryVAc* | 337 | 899 | 317 | 582 |

### Example 5 - Bioassay Results

Bioassays for nematode activity were performed as described in Example 3. PCR using the V7-ΔV8' primer pair was performed as described herein, for PS86Q3 which was bioassayed. The isolate was considered cryV PCR-positive since PCR using the V7-ΔV8' primer pair yielded an approximately 320-bp fragment. Nematode activity was found to be very highly correlated with a positive cryV PCR profile. C.e toxicity was found for PS86Q3.

## Claims

1. A method for controlling nematodes, which comprises contacting the nematodes with a nematicidal toxin obtainable from *Bacillus thuringiensis* isolate PS86Q3, or a nematicidal fragment of said toxin.

2. A method according to claim 1, wherein the toxin is 86Q3(a) or a nematicidal fragment thereof.

3. A method according to claim 1, wherein the toxin is 86Q3(a).

4. A method according to any preceding claim, wherein the toxin is produced by and present in a plant.

## Patentansprüche

1. Verfahren zur Bekämpfung von Nematoden, welches umfasst, dass man die Nematoden mit einem nematiziden Toxin, das aus *Bacillus thuringiensis*-Isolat PS86Q3 erhältlich ist, oder einem nematiziden Fragment des Toxins in Kontakt bringt.

2. Verfahren nach Anspruch 1, in dem das Toxin 86Q3(a) oder ein nematizides Fragment desselben ist.

3. Verfahren nach Anspruch 1, in dem das Toxin 86Q3(a) ist.

4. Verfahren nach irgendeinem vorangehenden Anspruch, in dem das Toxin von einer Pflanze produziert wird und darin vorhanden ist.

## Revendications

1. Procédé pour contrôler les nématodes, qui comprend la mise en contact des nématodes avec une toxine nématicide pouvant être obtenue à partir de l'isolat de *Bacillus thuringiensis* PS86Q3, ou un fragment nématicide de ladite toxine.

2. Procédé selon la revendication 1, dans lequel la toxine est 86Q3 (a) ou un fragment nématicide de celle-ci.

3. Procédé selon la revendication 1, dans lequel la toxine est 86Q3 (a).

4. Procédé selon l'une quelconque des revendications précédentes, dans lequel la toxine est produite par et est présente dans une plante.


### SEQUENCE LISTING

(1) GENERAL INFORMATION:
   (i) APPLICANT INFORMATION:
      - Applicant Name(s):: MYCOGEN CORPORATION
      - Street address:: 5501 Oberlin Drive
      - City:: San Diego
      - State/Province:: California
      - Country:: US
      - Postal code/zip:: 92121
      - Phone number:: (619) 453-8030 Fax number: (619)453-6991 Telex number:
   (ii) TITLE OF INVENTION: Identification of, and Uses For, Nematicidal Bacillus thuringiensis Genes, Toxins, and Isolates
   (iii) NUMBER OF SEQUENCES: 9
   (iv) CORRESPONDENCE ADDRESS:
      (A) ADDRESSEE: Saliwanchik & Saliwanchik
      (B) STREET: 2421 N.W. 41st Street, Suite A-1
      (C) CITY: Gainesville
      (D) STATE: Florida
      (E) COUNTRY: USA
      (F) ZIP: 32606
   (v) COMPUTER READABLE FORM:
      (A) MEDIUM TYPE: Floppy disk
      (B) COMPUTER: IBM PC compatible
      (C) OPERATING SYSTEM; PC-DOS/MS-DOS
      (D) SOFTWARE: PatentIn
   (vi) CURRENT APPLICATION DATA:
      (A) APPLICATION NUMBER: US
      (B) FILING DATE:
      (C) CLASSIFICATION:
   (vii) PRIOR APPLICATION DATA:
      (A) APPLICATION NUMBER: US 08/540,104
      (B) FILING DATE: 06-OCT-1995
      (C) CLASSIFICATION:
   (viii) ATTORNEY/AGENT INFORMATION:
      (A) NAME: Saliwanchik, David R.
      (B) REGISTRATION NUMBER: 31,794
      (C) REFERENCE/DOCKET NUMBER: MA94.C1
   (ix) TELECOMMUNICATION INFORMATION:
      (A) TELEPHONE: (352) 375-8100
      (B) TELEFAX: (352) 372-5800
(2) INFORMATION FOR SEQ ID NO:1:
   (i) SEQUENCE CHARACTERISTICS:
      (A) LENGTH: 20 bases
      (B) TYPE: nucleic acid
      (C) STRANDEDNESS: single
      (D) TOPOLOGY: linear
   (ii) MOLECULE TYPE: DNA (synthetic)
   (xi) SEQUENCE DESCRIPTION: SEQ ID NO:1:
   GATCGTMTWG ARTTTRTTCC 20
(2) INFORMATION FOR SEQ ID NO:2:
   (i) SEQUENCE CHARACTERISTICS:
      (A) LENGTH: 23 bases
      (B) TYPE: nucleic acid
      (C) STRANDEDNESS: single
      (D) TOPOLOGY: linear
   (ii) MOLECULE TYPE: DNA (synthetic)
   (xi) SEQUENCE DESCRIPTION: SEQ ID NO:2:
   AAAGTNGATG CMTTATCWGA TGA 23
(2) INFORMATION FOR SEQ ID NO:3:
   (i) SEQUENCE CHARACTERISTICS:
      (A) LENGTH: 20 bases
      (B) TYPE: nucleic acid
      (C) STRANDEDNESS: single
      (D) TOPOLOGY: linear
   (ii) MOLECULE TYPE: DNA (synthetic)
   (Xi) SEQUENCE DESCRIPTION: SEQ ID NO:3:
   ACACGTTATA HDGTTTCTGG 20
(2) INFORMATION FOR SEQ ID NO:4:
   (i) SEQUENCE CHARACTERISTICS:
      (A) LENGTH: 20 bases
      (B) TYPE: nucleic acid
      (C) STRANDEDNESS: single
      (D) TOPOLOGY: linear
   (ii) MOLECULE TYPE: DNA (synthetic)
   (xi) SEQUENCE DESCRIPTION: SEQ ID NO:4:
   TCATCWGATA AKGCATCNAC 20
(2) INFORMATION FOR SEQ ID NO:5:
   (i) SEQUENCE CHARACTERISTICS:
      (A) LENGTH: bases
      (B) TYPE: nucleic acid
      (C) STRANDEDNESS: single
      (D) TOPOLOGY: linear
   (ii) MOLECULE TYPE: DNA (synthetic)
   (xi) SEQUENCE DESCRIPTION: SEQ ID NO:5:
(2) INFORMATION FOR SEQ ID NO:6:
   (i) SEQUENCE CHARACTERISTICS:
      (A) LENGTH: 19 bases
      (B) TYPE: nucleic acid
      (C) STRANDEDNESS: single
      (D) TOPOLOGY: linear
   (ii) MOLECULE TYPE: DNA (synthetic)
   (xi) SEQUENCE DESCRIPTION: SEQ ID NO:6:
   CCGGAGGAAG GTGGGGATG 19
(2) INFORMATION FOR SEQ ID NO:7:
   (i) SEQUENCE CHARACTERISTICS:
      (A) LENGTH: 17 bases
      (B) TYPE: nucleic acid
      (C) STRANDEDNESS: single
      (D) TOPOLOGY: linear
   (ii) MOLECULE TYPE: DNA (synthetic)
   (xi) SEQUENCE DESCRIPTION: SEQ ID NO:7:
   CGATTACTAG CGATTCC 17
(2) INFORMATION FOR SEQ ID NO:8:
   (i) SEQUENCE CHARACTERISTICS:
      (A) LENGTH: 3504 base pairs
      (B) TYPE: nucleic acid
      (C) STRANDEDNESS: single
      (D) TOPOLOGY: linear
   (ii) MOLECULE TYPE: DNA (genomic)
   (vi) ORIGINAL SOURCE:
      (C) INDIVIDUAL ISOLATE: 167P
   (xi) SEQUENCE DESCRIPTION: SEQ ID NO:8:
   ATGACAAATC CAACTATACT ATATCCTAGT TACCATAATG TATTAGCTCA TCCGATTAGA    60
   TTAGATTCTT TTTTTGATCC ATTTGTAGAG ACATTTAAGG ATTTAAAAGG GGCTTGGGAA   120
   GAATTCGGAA AAACGGGATA TATGGACCCC TTAAAACAAC ACCTTCAAAT CGCATGGGAT   180
   ACTAGTCAAA ATGGAACAGT GGATTATTTA GCATTAACAA AAGCATCTAT ATCTCTCATA   240
   GGTTTAATTC CTGGTGCAGA CGCTGTAGTC CCTTTTATTA ATATGTTTGT AGACTTTATT   300
   TTTCCGAAAT TATTTGGAAG AGGTTCTCAA CAAAATGCTC AAGCTCAATT TTTCGAACTA   360
   ATCATAGAAA AAGTTAAAGA ACTTGTTGAT GAAGATTTTA GAAACTTTAC CCTTAATAAT   420
   CTACTCAATT ACCTTGATGG TATGCAAACA GCCTTATCAC ATTTCCAAAA CGATGTACAA   480
   ATTGCTATTT GTCAAGGAGA ACAACCAGGA CTTATGCTAG ATCAAACACC AACGGCTTGT   540
   ACTCCTACTA CAGACCATTT AATTTCTGTA AGAGAATCTT TTAAAGATGC TCGAACTACA   600
   ATTGAAACAG CTTTACCACA TTTTAAAAAT CCTATGCTAT CCACAAATGA TAACACTCCA   660
   GATTTTAATA GCGACACTGT CTTATTAACA TTACCAATGT ATACAACAGC AGCGACTTTA   720
   AATCTTATAT TACATCAAGG GTATATTCAA TTCGCAGAAA GATGGAAATC TGTAAATTAT   780
   GATGAAAGTT TTATAAATCA AACAAAAGTT GATTTGCAAC GTCGTATTCA GGACTATTCT   840
   ACTACTGTAT CTACCACTTT TGAAAAATTC AAACCTACTC TAAATCCATC AAATAAAGAA   900
   TCTGTTAATA AGTATAATAG ATATGTTCGT TCCATGACTC TTCAATCTTT AGACATTGCT   960
   GCAACATGGC CTACTTTAGA TAATGTTAAT TACCCTTCCA ATGTAGATAT TCAATTGGAT  1020
   CAAACTCGCT TAGTATTTTC AGATGTTGCA GGACCTTGGG AAGGTAATGA TAATATAACT  1080
   TCGAATATTA TAGATGTATT AACACCAATA AATACAGGGA TAGGATTTCA AGAAAGTTCA  1140
   GATCTTAGAA AATTCACTTA TCCACGAATA GAATTACAAA GCATGCAATT CCATGGACAA  1200
   TATGTAAACT CAAAAAGTGT AGAACATTGT TATAGCGATG GTCTTAAATT AAATTATAAA  1260
   AATAAAACTA TAACTGCAGG TGTAAGTAAT ATTGATGAAA GTAATCAAAA TAATAAACAT  1320
   AACTATGGTC CTGTAATAAA TAGTCCTATT ACTGATATCA ACGTAAATTC CCAAAATTCT  1380
   CAATATTTAG ATTTAAATTC AGTCATGGTA AATGGTGGTC AAAAAGTAGC CGGGTGTTCA  1440
   CCACTTAGTT CAAATGGTAA TTCTAATAAT GCTGCTTTAC CTAATCAAAA AATAAATGTT  1500
   ATTTATTCAG TACAATCAAA TGATAAACCA GAAAAACATG CAGACACTTA TAGAAAATGG  1560
   GGATATATGA GCAGTCATAT TCCTTATGAT CTTGTTCCAG AAAATGTAAT TGGAGATATA  1620
   GATCCGGATA CTAAACAACC GTCATTGCTT CTTAAAGGGT TTCCGGCAGA AAAAGGATAT  1680
   GGTGACTCAA TTGCATATGT ATCAGAACCT TTAAATGGTG CGAATGCAGT TAAACTTACT 1740
   TCATATCAAG TTCTCAAAAT GGAAGTTACA AATCAAACAA CTCAAAAATA TCGTATTCGC 1800
   ATACGTTATG CTACAGGTGG AGATACAGCT GCTTCTATAT GGTTTCATAT TATTGGTCCA 1860
   TCTGGAAATG ATTTAACAAA CGAAGGCCAT AACTTCTCTA GTGTATCTTC TAGAAATAAA 1920
   ATGTTTGTTC AGGGTAATAA CGGAAAATAT GTATTGAACA TCCTTACAGA TTCAATAGAA 1980
   TTACCATCAG GACAACAAAC TATTCTTATT CAAAATACTA ATTCTCAAGA TCTTTTTTTA 2040
   GATCGTATTG AATTTATTTC TCTCCCTTCT ACTTCTACTC CTACTTCTAC TAATTTTGTA 2100
   GAACCTGAAT CATTAGAAAA GATCATAAAC CAAGTTAATC AATTATTTAG CTCCTCATCT 2160
   CAAACTGAAT TGGCTCACAC TGTAAGCGAT TATAAAATTG ATCAAGTAGT GCTAAAAGTA 2220
   AATGCCTTAT CCGACGATGT ATTTGGTGTA GAGAAAAAAG CATTACGTAA ACTTGTGAAT 2280
   CAGGCCAAAC AACTCAGTAA AGCACGAAAT GTATTGGTCG GTGGAAACTT TGAAAAAGGT 2340
   CATGAATGGG CACTAAGCCG TGAAGCAACA ATGGTCGCAA ATCATGAGTT ATTCAAAGGG 2400
   GATCATTTAT TATTACCACC ACCAACCCTA TATCCATCGT ATGCATATCA AAAAATTGAT 2460
   GAATCGAAAT TAAAATCCAA TACACGTTAT ACTGTTTCCG GCTTTATTGC GCAAAGTGAA 2520
   CATCTAGAAG TCGTTGTGTC TCGATACGGG AAAGAAGTAC ATGACATGTT AGATATCCCG 2580
   TATGAAGAAG CCTTACCAAT TTCTTCTGAT GAGAGTCCAA ATTGTTGCAA ACCAGCTGCT 2640
   TGTCAGTGTT CATCTTGTGA TGGTAGTCAA TCAGATTCTC ATTTCTTTAG CTATAGTATC 2700
   GATGTTGGTT CCCTACAATC AGATGTAAAT CTCGGCATTG AATTCGGTCT TCGTATTGCG 2760
   AAACCAAACG GATTTGCGAA AATCAGTAAT CTAGAAATTA AAGAAGATCG TCCATTAACA 2820
   GAAAAAGAAA TCAAAAAAGT ACAACGTAAA GAACAAAAAT GGAAAAAAGC ATTTAACCAA 2880
   GAACAAGCCG AAGTAGCGAC AACACTCCAA CCAACGTTAG ATCAAATCAA TGCTTTGTAT 2940
   CAAAATGAAG ATTGGAACGG TTCCGTTCAC CCGCATGTGA CCTATCAACA TCTGTCCGCT 3000
   GTTGTTGTAC CAACGTTACC AAAACAAAGA CATTGGTTTA TGGAGGATCG AGAAGGCGAA 3060
   CATGTTGTTC TGACGCAACA ATTCCAACAA GCATTGGATC GTGCGTTCCA ACAAATCGAA 3120
   GAACAAAACT TAATCCACAA TGGTAATTTT GCGAATGGAT TAACAGATTG GACTGTCACA 3180
   GGAGATGCAC AACTTACGAT CTTTGACGAA GATCCAGTAT TAGAACTAGC GCATTGGGAT 3240
   GCAAGTATCT CTCAAACCAT TGAAATTATG GATTTTGAAG AAGACACAGA ATACAAACTG 3300
   CGTGTACGTG GAAAAGGCAA AGGAACAGTT ACCGTTCAAC ATGGAGAAGA AGAATTAGAA 3360
   ACGATGACAT TCAATACAAC GAGTTTTACA ACACAAGAAC AAACCTTCTA CTTCGAAGGA 3420
   GATACAGTGG ACGTACATGT TCAATCAGAG AATAACACAT TCCTGATAGA TAGTGTGGAA 3480
   CTCATTGAAA TCATAGAAGA GTAA 3504
(2) INFORMATION FOR SEQ ID NO:9:
   (i) SEQUENCE CHARACTERISTICS:
      (A) LENGTH: 1168 amino acids
      (B) TYPE: amino acid
      (C) STRANDEDNESS: single
      (D) TOPOLOGY: linear
   (ii) MOLECULE TYPE: peptide
   (vi) ORIGINAL SOURCE:
      (C) INDIVIDUAL ISOLATE: 167p
   (xi) SEQUENCE DESCRIPTION: SEQ ID NO:9:
   Met Thr Asn Pro Thr Ile Leu Tyr Pro Ser Tyr His Asn Val Leu Ala
   1               5                   10                  15
   His Pro Ile Arg Leu Asp Ser Phe Phe Asp Pro Phe Val Glu Thr Phe
               20                  25                  30
   Lys Asp Leu Lys Gly Ala Trp Glu Glu Phe Gly Lys Thr Gly Tyr Met
           35                  40                  45
   Asp Pro Leu Lys Gln His Leu Gln Ile Ala Trp Asp Thr Ser Gln Asn
       50                  55                  60
   Gly Thr Val Asp Tyr Leu Ala Leu Thr Lys Ala Ser Ile Ser Leu Ile
   65                  70                  75                  80
   Gly Leu Ile Pro Gly Ala Asp Ala Val Val Pro Phe Ile Asn Met Phe
                   85                  90                  95
   Val Asp Phe Ile Phe Pro Lys Leu Phe Gly Arg Gly Ser Gln Gln Asn
               100                 105                 110
   Ala Gln Ala Gln Phe Phe Glu Leu Ile Ile Glu Lys Val Lys Glu Leu
           115                 120                 125
   Val Asp Glu Asp Phe Arg Asn Phe Thr Leu Asn Asn Leu Leu Asn Tyr
       130                 135                 140
   Leu Asp Gly Met Gln Thr Ala Leu Ser His Phe Gln Asn Asp Val Gln
   145                 150                 155                 160
   Ile Ala Ile Cys Gln Gly Glu Gln Pro Gly Leu Met Leu Asp Gln Thr
                   165                 170                 175
   Pro Thr Ala Cys Thr Pro Thr Thr Asp His Leu Ile Ser Val Arg Glu
               180                 185                 190
   Ser Phe Lys Asp Ala Arg Thr Thr Ile Glu Thr Ala Leu Pro His Phe
           195                 200                 205
   Lys Asn Pro Met Leu Ser Thr Asn Asp Asn Thr Pro Asp Phe Asn Ser
       210                 215                 220
   Asp Thr Val Leu Leu Thr Leu Pro Met Tyr Thr Thr Ala Ala Thr Leu
   225                 230                 235                 240
   Asn Leu Ile Leu His Gln Gly Tyr Ile Gln Phe Ala Glu Arg Trp Lys
                   245                 250                 255
   Ser Val Asn Tyr Asp Glu Ser Phe Ile Asn Gln Thr Lys Val Asp Leu
               260                 265                 270
   Gln Arg Arg Ile Gln Asp Tyr Ser Thr Thr Val Ser Thr Thr Phe Glu
           275                 280                 285
   Lys Phe Lys Pro Thr Leu Asn Pro Ser Asn Lys Glu Ser Val Asn Lys
       290                 295                 300
   Tyr Asn Arg Tyr Val Arg Ser Met Thr Leu Gln Ser Leu Asp Ile Ala
   305                 310                 315                 320
   Ala Thr Trp Pro Thr Leu Asp Asn Val Asn Tyr Pro Ser Asn Val Asp
                   325                 330                 335
   Ile Gln Leu Asp Gln Thr Arg Leu Val Phe Ser Asp Val Ala Gly Pro
               340                 345                 350
   Trp Glu Gly Asn Asp Asn Ile Thr Ser Asn Ile Ile Asp Val Leu Thr
           355                 360                 365
   Pro Ile Asn Thr Gly Ile Gly Phe Gln Glu Ser Ser Asp Leu Arg Lys
       370                 375                 380
   Phe Thr Tyr Pro Arg Ile Glu Leu Gln Ser Met Gln Phe His Gly Gln
   385                 390                 395                 400
   Tyr Val Asn Ser Lys Ser Val Glu His Cys Tyr Ser Asp Gly Leu Lys
                   405                 410                 415
   Leu Asn Tyr Lys Asn Lys Thr Ile Thr Ala Gly Val Ser Asn Ile Asp
               420                 425                 430
   Glu Ser Asn Gln Asn Asn Lys His Asn Tyr Gly Pro Val Ile Asn Ser
           435                 440                 445
   Pro Ile Thr Asp Ile Asn Val Asn Ser Gln Asn Ser Gln Tyr Leu Asp
       450                 455                 460
   Leu Asn Ser Val Met Val Asn Gly Gly Gln Lys Val Ala Gly Cys Ser
   465                 470                 475                 480
   Pro Leu Ser Ser Asn Gly Asn Ser Asn Asn Ala Ala Leu Pro Asn Gln
                   485                 490                 495
   Lys Ile Asn Val Ile Tyr Ser Val Gln Ser Asn Asp Lys Pro Glu Lys
               500                 505                 510
   His Ala Asp Thr Tyr Arg Lys Trp Gly Tyr Met Ser Ser His Ile Pro
           515                 520                 525
   Tyr Asp Leu Val Pro Glu Asn Val Ile Gly Asp Ile Asp Pro Asp Thr
       530                 535                 540
   Lys Gln Pro Ser Leu Leu Leu Lys Gly Phe Pro Ala Glu Lys Gly Tyr
   545                 550                 555                 560
   Gly Asp Ser Ile Ala Tyr Val Ser Glu Pro Leu Asn Gly Ala Asn Ala
                   565                 570                 575
   Val Lys Leu Thr Ser Tyr Gln Val Leu Lys Met Glu Val Thr Asn Gln
               580                 585                 590
   Thr Thr Gln Lys Tyr Arg Ile Arg Ile Arg Tyr Ala Thr Gly Gly Asp
           595                 600                 605
   Thr Ala Ala Ser Ile Trp Phe His Ile Ile Gly Pro Ser Gly Asn Asp
       610                 615                 620
   Leu Thr Asn Glu Gly His Asn Phe Ser Ser Val Ser Ser Arg Asn Lys
   625                 630                 635                 640
   Met Phe Val Gln Gly Asn Asn Gly Lys Tyr Val Leu Asn Ile Leu Thr
                   645                 650                 655
   Asp Ser Ile Glu Leu Pro Ser Gly Gln Gln Thr Ile Leu Ile Gln Asn
               660                 665                 670
   Thr Asn Ser Gln Asp Leu Phe Leu Asp Arg Ile Glu Phe Ile Ser Leu
           675                 680                 685
   Pro Ser Thr Ser Thr Pro Thr Ser Thr Asn Phe Val Glu Pro Glu Ser
       690                 695                 700
   Leu Glu Lys Ile Ile Asn Gln Val Asn Gln Leu Phe Ser Ser Ser Ser
   705                 710                 715                 720
   Gln Thr Glu Leu Ala His Thr Val Ser Asp Tyr Lys Ile Asp Gln Val
                   725                 730                 735
   Val Leu Lys Val Asn Ala Leu Ser Asp Asp Val Phe Gly Val Glu Lys
               740                 745                 750
   Lys Ala Leu Arg Lys Leu Val Asn Gln Ala Lys Gln Leu Ser Lys Ala
           755                 760                 765
   Arg Asn Val Leu Val Gly Gly Asn Phe Glu Lys Gly His Glu Trp Ala
       770                 775                 780
   Leu Ser Arg Glu Ala Thr Met Val Ala Asn His Glu Leu Phe Lys Gly
   785                 790                 795                 800
   Asp His Leu Leu Leu Pro Pro Pro Thr Leu Tyr Pro Ser Tyr Ala Tyr
   805 810 815
   Gln Lys Ile Asp Glu Ser Lys Leu Lys Ser Asn Thr Arg Tyr Thr Val
   820 825 830
   Ser Gly Phe Ile Ala Gln Ser Glu His Leu Glu Val Val Val Ser Arg
   835 840 845
   Tyr Gly Lys Glu Val His Asp Met Leu Asp Ile Pro Tyr Glu Glu Ala
   850 855 860
   Leu Pro Ile Ser Ser Asp Glu Ser Pro Asn Cys Cys Lys Pro Ala Ala
   865 870 875 880
   Cys Gln Cys Ser Ser Cys Asp Gly Ser Gln Ser Asp Ser His Phe Phe
   885 890 895
   Ser Tyr Ser Ile Asp Val Gly Ser Leu Gln Ser Asp Val Asn Leu Gly
   900 905 910
   Ile Glu Phe Gly Leu Arg Ile Ala Lys Pro Asn Gly Phe Ala Lys Ile
   915 920 925
   Ser Asn Leu Glu Ile Lys Glu Asp Arg Pro Leu Thr Glu Lys Glu Ile
   930 935 940
   Lys Lys Val Gln Arg Lys Glu Gln Lys Trp Lys Lys Ala Phe Asn Gln
   945 950 955 960
   Glu Gln Ala Glu Val Ala Thr Thr Leu Gln Pro Thr Leu Asp Gln Ile
   965 970 975
   Asn Ala Leu Tyr Gln Asn Glu Asp Trp Asn Gly Ser Val His Pro His
   980 985 990
   Val Thr Tyr Gln His Leu Ser Ala Val Val Val Pro Thr Leu Pro Lys
   995 1000 1005
   Gln Arg His Trp Phe Met Glu Asp Arg Glu Gly Glu His Val Val Leu
   1010 1015 1020
   Thr Gln Gln Phe Gln Gln Ala Leu Asp Arg Ala Phe Gln Gln Ile Glu
   1025 1030 1035 1040
   Glu Gln Asn Leu Ile His Asn Gly Asn Phe Ala Asn Gly Leu Thr Asp
   1045 1050 1055
   Trp Thr Val Thr Gly Asp Ala Gln Leu Thr Ile Phe Asp Glu Asp Pro
   1060 1065 1070
   Val Leu Glu Leu Ala His Trp Asp Ala Ser Ile Ser Gln Thr Ile Glu
   1075 1080 1085
   Ile Met Asp Phe Glu Glu Asp Thr Glu Tyr Lys Leu Arg Val Arg Gly
   1090 1095 1100
   Lys Gly Lys Gly Thr Val Thr Val Gln His Gly Glu Glu Glu Leu Glu
   1105                1110                1115                1120
   Thr Met Thr Phe Asn Thr Thr Ser Phe Thr Thr Gln Glu Gln Thr Phe
                   1125                1130                1135
   Tyr Phe Glu Gly Asp Thr Val Asp Val His Val Gln Ser Glu Asn Asn
               1140                1145                1150
   Thr Phe Leu Ile Asp Ser Val Glu Leu Ile Glu Ile Ile Glu Glu Met
           1155                1160                1165